# EUROPEAN PATENT APPLICATION

(11) **EP 1 361 270 A2**
(43) Date of publication of application: **12.11.2003**
(21) Application number: 03251577.7
(22) Date of filing: 14.03.2003
(51) Int. Cl.: C12N 5/10, A01K 67/027, C12Q 1/68

(54) **Disruption of the REDK gene**

(30) Priority: 30.03.2002 US 368810
(71) Applicant: Pfizer Products Inc., Groton, Connecticut 06340 (US)
(72) Inventor: Allen, Melanie Ruth, Groton, Connecticut 06340 (US); Brissette, William Howard, Groton, Connecticut 06340 (US); Hambor, John Edward, Groton, Connecticut 06340 (US); Neote, Kuldeep Singh, Groton, Connecticut 06340 (US); Roach, Marsha Lynn, Groton, Connecticut 06340 (US)
(74) Representative: Walls, Alan James

(57) **Abstract**

The invention features non-human mammals and animal cells that contain a targeted disruption of a REDK gene.

SEQUENCE LISTING

<110>  PFIZER PRODUCTS INC

<120>  DISRUPTION OF THE REDK GENE

<130>  PC23222A

<150>  US 60/368,810
<151>  2002-03-30

<160>  4

<170>  PatentIn version 3.1

<210>  1
<211>  21
<212>  DNA
<213>  Mouse

<400>  1
gccagctcat tcctccactc a                                                21


<210>  2
<211>  22
<212>  DNA
<213>  Mouse

<400>  2
atttctttga gacagagact gc                                               22


<210>  3
<211>  20
<212>  DNA
<213>  Mouse

<400>  3
aggtcatcga ctttggctcc                                                  20


<210>  4
<211>  21
<212>  DNA
<213>  Mouse

<400>  4
cactagctaa tcagctttgg c                                                21

## Description

### Field of the Invention

The present invention features genetically-modified non-human mammals and animal cells having a disrupted REDK gene.

### Background of the Invention

Anemia is a condition wherein the oxygen-transporting capacity of blood decreases, which results, for example, from a decrease in red blood cell concentration. Anemia can lead to impaired physical activity, organ failure, and, ultimately, death.

Regulatory erythroid kinase (REDK) appears to play a role in erythropoiesis. In an *in vitro* colony assay, inhibition of REDK by antisense oligonucleotides in human CD34+ hematopoietic stem cells has been shown to increase the frequency of the erythroid blast-forming units (BFU-Es) and erythroid stage colony forming units (CFU-Es). Lord, et al. (2000). These results suggest that REDK is involved in the commitment of pluripotent stem cells to the erythroid lineage. Alternatively, REDK may act as an inhibitor of erythropoiesis.

REDK belongs to the mammalian DYRK family of kinases. Like other members of the DYRK family, REDK is believed to be a dual specificity kinase catalyzing phosphorylation at serine/threonine residues and autophosphorylation at tyrosine residues (see Lord, et al. (2000); and Kentrup, Heiner, et al. (1996)).

cDNA sequences of human REDK are disclosed in Genbank accession numbers Y12735, AF186773 and AF186774. The mouse REDK cDNA sequence is disclosed in GenBank Accession BC006704.

Therapies that modulate the function of REDK may provide a means for the treatment of conditions related to erythropoiesis, such as anemia.
Genetically-modified mammals and cells having a disrupted REDK gene, may be valuable for defining the physiological role of REDK and for understanding the therapeutic implications associated with modulating REDK activity.

### Summary of the Invention

One aspect of this invention provides genetically-modified, non-human mammals, or progeny mammals thereof, wherein the modification comprises a disrupted REDK gene.

In a preferred embodiment, said mammals or progeny mammals thereof are rodents, preferably mice. In another preferred embodiment, said mammals or progeny mammals thereof, are homozygous for said modification.

Another aspect of this invention features genetically-modified animal cells, or progeny cells thereof, wherein the modification comprises a disrupted REDK gene.

In a preferred embodiment, said animal cells, or progeny cells thereof, are embryonic stem (ES) cells or ES-like cells. In another preferred embodiment, said cells, or progeny cells thereof, are isolated from a genetically-modified, non-human mammal, or a progeny mammal thereof, containing a modification that results in a disrupted REDK gene. In a further preferred embodiment, said cells, or progeny cells thereof, are hematopoietic cells or testicular cells. In an additional embodiment, said cells, or progeny cells thereof, are mammalian, preferably, of murine or human origin. In a still further preferred embodiment, said cells, or progeny cells thereof are homozygous for said modification.

In an additional aspect, the invention features method of identifying a biological characteristic associated with reduction or elimination of REDK activity. The method involves comparing a biological characteristic of a genetically-modified, non-human mammal or animal cell comprising a disrupted REDK gene to the characteristic of the appropriate wild-type control. Preferably, the biological characteristic is selected from a morphological, histological, metabolic, developmental, or behavioral characteristic.

An additional aspect of this invention provides methods of identifying a gene that demonstrates modified expression as a result of reduced REDK activity in an animal cell, comprising comparing the expression of at least one gene other than REDK, in a cell of this invention and a wild-type animal cell. In a preferred embodiment, said expression profile is prepared using a microarray.

Another aspect of this invention features methods of identifying a protein that demonstrates a modified level or post-translational processing as a result of reduced REDK activity in an animal cell, comprising comparing the proteomic profile of a cell of this invention to that from a wild-type animal cell.

Those skilled in the art will fully understand the terms used herein in the description and the appendant claims to describe the present invention. Nonetheless, unless otherwise provided herein, the following terms are as described immediately below.

A "disrupted REDK gene" refers to a REDK gene that is genetically-modified such that the cellular activity of the REDK polypeptide encoded by the disrupted gene is decreased or, preferably, eliminated in cells that normally express a wild type version of the REDK gene. When the genetic modification effectively eliminates all wild type copies of the REDK gene in a cell (e.g., the genetically-modified, non-human mammal or animal cell is homozygous for the REDK gene disruption or the only wild type copy of the REDK gene originally present is now disrupted), the genetic modification results in a reduction in REDK polypeptide activity as compared to a control cell that expresses the wild type REDK gene. This reduction in REDK polypeptide activity results from either reduced REDK gene expression (i.e., REDK mRNA levels are effectively reduced resulting in reduced levels of REDK polypeptide) and/or because the disrupted REDK gene encodes a mutated polypeptide with altered, e.g., reduced, function as compared to a wild type REDK polypeptide. Preferably, the activity of REDK polypeptide in the genetically-modified, non-human mammal or animal cell is reduced to 50% or less of wild type levels, more preferably, to 25% or less, and, even more preferably, to 10% or less of wild type levels. Most preferably, the REDK gene disruption results in non-detectable REDK activity.

A "genetically-modified, non-human mammal" containing a disrupted REDK gene refers to a non-human mammal created by genetic engineering to contain a disrupted REDK gene, as well as a progeny of such non-human mammal that inherits the disrupted REDK gene. A genetically-modified non-human mammal may be produced, for example, by creating a blastocyst or embryo carrying the desired genetic modification and then implanting the blastocyst or embryo in a foster mother for in utero development. The genetically-modified blastocyst or embryo can be made, in the case of mice, by implanting a genetically-modified embryonic stem (ES) cell into a mouse blastocyst or by aggregating ES cells with tetraploid embryos. Alternatively, various species of genetically-modified embryos can be obtained by nuclear transfer. In the case of nuclear transfer, the donor cell is a somatic cell or a pluripotent stem cell, and it is engineered to contain the desired genetic modification that disrupts the REDK gene. The nucleus of this cell is then transferred into a fertilized or parthenogenetic oocyte that is enucleated; the resultant embryo is reconstituted and developed into a blastocyst. A genetically-modified blastocyst produced by either of the above methods is then implanted into a foster mother according to standard methods well known to those skilled in the art. A "genetically-modified, non-human mammal" includes all progeny of the non-human mammals created by the methods described above, provided that the progeny inherit at least one copy of the genetic modification that disrupts the REDK gene. It is preferred that all somatic cells and germline cells of the genetically-modified non-human mammal contain the modification. Preferred non-human mammals that are genetically-modified to contain a disrupted REDK gene include rodents, such as mice and rats, cats, dogs, rabbits, guinea pigs, hamsters, sheep, pigs, and ferrets.

A "genetically-modified animal cell" containing a disrupted REDK gene refers to an animal cell (preferably a mammalian cell), including a human cell, created by genetic engineering to contain a disrupted REDK gene, as well as daughter cells that inherit the disrupted REDK gene. These cells may be genetically-modified in culture according to any standard method known in the art. As an alternative to genetically modifying the cells in culture, non-human mammalian cells may also be isolated from a genetically-modified, non-human mammal that contains a REDK gene disruption. The animal cells of the invention may be obtained from primary cell or tissue preparations as well as culture-adapted, tumorigenic, or transformed cell lines. These cells and cell lines are derived, for example, from endothelial cells, epithelial cells, islets, neurons and other neural tissue-derived cells, mesothelial cells, osteocytes, lymphocytes, chondrocytes, hematopoietic cells, immune cells, cells of the major glands or organs (e.g., testicle, liver, lung, heart, stomach, pancreas, kidney, and skin), muscle cells (including cells from skeletal muscle, smooth muscle, and cardiac muscle), exocrine or endocrine cells, fibroblasts, and embryonic and other totipotent or pluripotent stem cells (e.g., ES cells, ES-like cells, embryonic germline cells, and other stem cells, such as progenitor cells and tissue-derived stem cells). The preferred genetically-modified cells are ES cells, more preferably, mouse or rat ES cells, and, most preferably, human ES cells.

A non-human mammal or a animal cell that is "genetically-modified" is heterozygous or homozygous for a modification that is introduced into the non-human mammal or animal cell, or into a progenitor non-human mammal or animal cell, by genetic engineering. The standard methods of genetic engineering that are available for introducing the modification include homologous recombination, viral vector gene trapping, irradiation, chemical mutagenesis, and the transgenic expression of a nucleotide sequence encoding antisense RNA alone or in combination with catalytic ribozymes. Preferred methods for genetic modification to disrupt a gene are those which modify an endogenous gene by inserting a "foreign nucleic acid sequence" into the gene locus, e.g., by homologous recombination or viral vector gene trapping. A "foreign nucleic acid sequence" is an exogenous sequence that is non-naturally occurring in the gene. This insertion of foreign DNA can occur within any region of the REDK gene, e.g., in an enhancer, promoter, regulator region, noncoding region, coding region, intron, or exon. The most preferred method of genetic engineering for gene disruption is homologous recombination, in which the foreign nucleic acid sequence is inserted in a targeted manner either alone or in combination with a deletion of a portion of the endogenous gene sequence.

"Homozygosity", when referring to REDK gene disruption in a non-human mammal or an animal cell, means a non-human mammal or animal cell having all alleles of the REDK gene that are disrupted. However, the REDK sequences of each of these alleles need not be identical. For example, a non-human mammal may be homozygous for REDK disruption wherein one allele of REDK is disrupted as a result of deletion of one region of the gene sequence and the other allele is disrupted as a result of deletion of another region of the gene sequence.

"ES cell" or an "ES-like cell" means a pluripotent stem cell derived from an embryo, from a primordial germ cell, or from a teratocarcinoma, that is capable of indefinite self-renewal as well as differentiation into cell types that are representative of all three embryonic germ layers.

"Microarray" means an arrangement of distinct polynucleotides on a substrate, as more fully described herein.

"Reduced REDK activity" means a decrease in the activity of the REDK enzyme as a result of genetic manipulation of the REDK gene that causes a reduction in the level of functional REDK polypeptide in a cell, or as the result of administration of a pharmacological agent that inhibits REDK activity.

"Wild-type", when referring to a non-human mammal or a animal cell, means a non-human mammal or an animal cell, as the case may be, that does not comprise a disrupted REDK gene. For example, in a comparison of a particular characteristic of a non-human mammal of this invention to that characteristic in a wild-type mammal, the term wild-type refers to non-human mammal that does not comprise a disrupted REDK gene (i.e., a mammal whose REDK gene is wild-type). Preferably, a wild-type non-human mammal is substantially similar, and, more preferably, substantially identical, to a non-human mammal of the invention, except for the non-disruption or disruption of the REDK gene, respectively. Likewise, for example, in a comparison of a particular characteristic of an animal cell of this invention to that characteristic in a wild-type mammal cell, the term wild-type refers to an animal cell that does not comprise a disrupted REDK gene (i.e., a cell whose REDK gene is wild-type). Preferably, a wild-type animal cell is substantially similar, and, more preferably, substantially identical, to an animal cell of the invention, except for the non-disruption or disruption of the REDK gene, respectively. "Wild-type" may also refer to a REDK gene allele that is not disrupted, as, for example, in an animal cell or non-human mammal having one disrupted REDK gene allele and one wild-type REDK gene allele (i.e., heterozygous for REDK disruption).

Other features and advantages of the invention will be apparent from the following detailed description and from the claims. While the invention is described in connection with specific embodiments, it will be understood that other changes and modifications that may be practiced are also part of this invention and are also within the scope of the appendant claims. This application is intended to cover any equivalents, variations, uses, or adaptations of the invention that follow, in general, the principles of the invention, including departures from the present disclosure that come within known or customary practice within the art, and that are able to be ascertained without undue experimentation. Additional guidance with respect to making and using nucleic acids and polypeptides is found in standard textbooks of molecular biology, protein science, and immunology (see, e.g., Davis et al. (1986); Hames et al.(1985); Sambrook, J. et al. (1989); Ausubel, F.M. et al. (2001); Dracopoli, et al. (1994); Coligan, John E., et al. (2002); and Coligan, John E., et al. (1994). All publications, including published patent applications and issued patents, mentioned herein are incorporated by reference in their entireties.

### ABBREVIATIONS

The following abbreviations are used herein:
"BFU-E" means erythroid blast-forming unit
"cDNA" means complimentary DNA
"CFU-E" means erythroid stage colony forming unit
"DMEM" means Dulbecco's Modified Eagle's Medium
"DNA" means deoxyribonucleic acid
"IRES" independent ribosome entry site
"LIF" leukemia inhibitory factor
"MEM" means Modified Eagle's Medium
"nm" means nanometer
"nM" means nanomolar
"PCR" means polymerase chain reaction
"PEF" means primary embryonic fibroblast
"REDK" means regulatory erythroid kinase
"RFLP" means restriction fragment length polymorphism
"RNA" means ribonucleic acid
"mRNA" means messenger RNA

### Brief Description of the Drawings

Figure 1A is a schematic depicting the backbone vector, pJNS₂. Figure 1B depicts the gene targeting vector of the present invention.
Figures 2A and 2B show the location of homologous recombination of the vector in the endogenous murine REDK gene.
Figure 3 is a Southern blot of PCR-based genotyping of REDK (+/-) and REDK (-/-) mice.
Figure 4 compares the level of erythroid colony formation of REDK disrupted ES cells and wild type ES cells.

### Detailed Description of the Invention

The genetically-modified, non-human mammals and genetically-modified animal cells, including human cells, of the invention are heterozygous or homozygous for a modification that disrupts the REDK gene. The animal cells may be derived by genetically engineering cells in culture, or, in the case of non-human mammalian cells, the cells may be isolated from genetically-modified, non-human mammals.

### Disruption of the REDK Gene

In order to create genetically-modified non-human mammals and mammal cells of the invention, the REDK gene locus may be disrupted using techniques for genetic modification known in the art, including chemical mutagenesis (Rinchik (1991); Russell (1994)), irradiation (Russell, (1994)), transgenic expression of REDK gene antisense RNA, either alone or in combination with a catalytic RNA ribozyme sequence (Luyckx et al. (1999); Sokol et al., (1996); Efrat et al., (1994); Larsson et al. (1994)) and, as further discussed below, the disruption of the REDK gene by the insertion of a foreign nucleic acid sequence into the REDK gene locus. Preferably, the REDK gene is disrupted by insertion of a foreign nucleic acid sequence, more preferably by means of homologous recombination or by the insertion of a viral vector. Even more preferably, the method of REDK gene disruption is homologous recombination and includes a deletion of a portion of the endogenous REDK gene sequence.

The integration of the foreign sequence disrupts the REDK gene through one or more of the following mechanisms: by interfering with the REDK gene transcription or translation process (e.g., by interfering with promoter recognition, or by introducing a transcription termination site or a translational stop codon into the REDK gene); by distorting the REDK gene coding sequence such that it no longer encodes a REDK polypeptide with normal function (e.g., by inserting a foreign coding sequence into the REDK gene coding sequence, by introducing a frameshift mutation or amino acid(s) substitution; or, in the case of a double crossover event, by deleting a portion of the REDK gene coding sequence that is required for expression of a functional REDK protein).

To insert a foreign sequence into a REDK gene locus in the genome of a cell to create the genetically-modified non-human mammals and mammal cells of the invention, the foreign DNA sequence is introduced into the cell according to a standard method known in the art such as electroporation, calcium-phosphate precipitation, retroviral infection, microinjection, biolistics, liposome transfection, DEAE-dextran transfection, or transferrinfection (see, e.g., Neumann et al. (1982); Potter et al. (1984); Chu et al. (1987); Thomas and Capecchi (1987); Baum et al. (1994); Biewenga et al., (1997); Zhang et al., (1993); Ray and Gage (1992); Lo (1983); Nickoloff et al. (1998); Linney et al. (1999); Zimmer and Gruss, (1989); and Robertson et al., (1986). A preferred method in the practice of the present invention for introducing foreign DNA into a cell is electroporation.

### 1. Homologous Recombination

The method of homologous recombination targets the REDK gene for disruption by introducing a REDK gene targeting vector into a cell containing a REDK gene. The vector targets the REDK gene using a nucleotide sequence in the vector that is homologous to the REDK gene. This homologous region facilitates hybridization between the vector and the endogenous sequence of the REDK gene. Upon hybridization, the probability of a crossover event between the targeting vector and genomic sequences increases greatly. Such a crossover event results in the integration of the vector sequence into the REDK gene locus and the likely functional disruption of the REDK gene.

General principles regarding the construction of vectors used for targeting are reviewed in Bradley et al. (1992). Two types of vectors that may be used to insert DNA by homologous recombination are insertion vectors and replacement vectors. The preferred vectors for preparing the genetically-modified non-human mammals and mammal cells of the invention by homologous recombination are replacement vectors

Insertion vectors are a circular DNA molecule which include a region of REDK gene homology with a double stranded break. Following hybridization between the homology region and the endogenous REDK gene, a single crossover event at the double stranded break results in the insertion of the entire vector sequence into the endogenous gene at the site of crossover.

A replacement vector is colinear rather than circular. Replacement vector integration into the REDK gene requires a double crossover event, i.e. crossing over at two sites of hybridization between the targeting vector and the REDK gene. This double crossover event results in the integration of a vector sequence that is sandwiched between the two sites of crossover into the REDK gene and the deletion of the corresponding endogenous REDK gene sequence that originally spanned between the two sites of crossover (see, for example, Thomas and Capecchi (1987); Mansour et al. (1988); Mansour et al. (1990); and Mansour (1990)).

A region of homology in a targeting vector used to create the genetically-modified non-human mammals and animal cells of the invention is generally at least 100 nucleotides in length. Most preferably, the homology region is at least 1-5 kilobases (kb) in length. Although there is no demonstrated minimum length or minimum degree of relatedness required for a homology region, targeting efficiency for homologous recombination generally corresponds with the length and the degree of relatedness between the targeting vector and the REDK gene locus. In the case where a replacement vector is used, and a portion of the endogenous REDK gene is deleted upon homologous recombination, an additional consideration is the size of the deleted portion of the endogenous REDK gene. If this portion of the endogenous REDK gene is greater than 1 kb in length, then a targeting cassette with regions of homology that are longer than 1 kb is recommended to enhance the efficiency of recombination. Further guidance regarding the selection and use of sequences effective for homologous recombination, based on the present description, is described in the literature (see, for example, Deng and Capecchi (1992); Bollag et al. (1989); and Waldman and Liskay (1988)).

As those skilled in the art will recognize based upon the present invention, a wide variety of cloning vectors may be used as vector backbones in the construction of the REDK gene targeting vectors of the present invention, including pBluescript-related plasmids (e.g., Bluescript KS+11), pQE70, pQE60, pQE-9, pBS, pD10, phagescript, phiX174, pBK Phagemid, pNH8A, pNH16a, pNH18Z, pNH46A, ptrc99a, pKK223-3, pKK233-3, pDR540, and pRIT5 PWLNEO, pSV2CAT, pXT1, pSG (Stratagene), pSVK3, PBPV, PMSG, and pSVL, pBR322 and pBR322-based vectors, pMB9, pBR325, pKH47, pBR328, pHC79, phage Charon 28, pKB11, pKSV-10, pK19 related plasmids, pUC plasmids, and the pGEM series of plasmids. These vectors are available from a variety of commercial sources (e.g., Boehringer Mannheim Biochemicals, Indianapolis, IN; Qiagen, Valencia, CA; Stratagene, La Jolla, CA; Promega, Madison, WI; and New England Biolabs, Beverly, MA). However, any other vectors, e.g. plasmids, viruses, or parts thereof, may be used as long as they are replicable and viable in the desired host. The vector may also comprise sequences which enable it to replicate in the host whose genome is to be modified. The use of such a vector can expand the interaction period during which recombination can occur, increasing the efficiency of targeting (see Ausubel et al (2001), Unit 9.16, Fig. 9.16.1).

The specific host employed for propagating the targeting vectors of the present invention is not critical. Examples include *E. coli* K12 RR1 (Bolivar et al., (1977)), *E. coli* K12 HB101 (ATCC No. 33694), *E. coli* MM21 (ATCC No. 336780), *E. coli* DH1 (ATCC No. 33849), *E. coli* strain DH5α, and *E. coli* STBL2. Alternatively, hosts such as *C. cerevisiae* or *B. subtilis* can be used. The above-mentioned hosts are available commercially (e.g., Stratagene, La Jolla, CA; and Life Technologies, Rockville, MD).

In order to create a targeting vector, a REDK gene targeting construct is added to a vector backbone, such as, for example, a vector backbone described above. The REDK gene targeting constructs of the invention have at least one REDK gene homology region.

Preferably, the targeting constructs of the invention also include an exogenous nucleotide sequence encoding a positive marker protein. The stable expression of a positive marker after vector integration confers an identifiable characteristic on the cell, ideally, without compromising cell viability. Therefore, in the case of a replacement vector, the marker gene is positioned between two flanking homology regions so that it integrates into the REDK gene following the double crossover event in a manner such that the marker gene is positioned for expression after integration.

It is preferred that the positive marker protein confer a selectable phenotypic characteristic, such as, for example, a characteristic that enhances the survival of the cell under otherwise lethal conditions. Thus, by imposing the selectable condition, one may isolate cells that stably express the positive selectable marker-encoding vector sequence from other cells that have not successfully integrated the vector sequence on the basis of viability. Examples of positive selectable marker proteins (and their agents of selection) include neo (G418 or kanomycin), hyg (hygromycin), hisD (histidinol), gpt (xanthine), ble (bleomycin), and hprt (hypoxanthine) (see, for example, U.S. Pat. No. 5,464,764, and Capecchi (1989)). Other positive markers that may also be used as an alternative to a selectable marker include reporter proteins such as β-galactosidase, firefly luciferase, or GFP (see, for example, Robinson, J. Paul (1997), Unit 9.5, and Ausubel, F.M. et al. (2001), Unit 9.6).

The above-described positive selection step does not distinguish between cells that have integrated the vector by targeted homologous recombination at the REDK gene locus versus random, non-homologous integration of vector sequence into any chromosomal position. Therefore, when using a replacement vector for homologous recombination to make the genetically-modified non-human mammals and animal cells of the invention, it is also preferred to include a nucleotide sequence encoding a negative selectable marker protein. Expression of a negative selectable marker causes a cell expressing the marker to lose viability when exposed to a certain agent (i.e., the marker protein becomes lethal to the cell under certain selectable conditions). Examples of negative selectable markers (and their agents of lethality) include herpes simplex virus thymidine kinase (gancyclovir or 1,2-deoxy-2-fluoro-a-d-arabinofuransyl-5-iodouracil), Hprt (6-thioguanine or 6-thioxanthine), and diphtheria toxin, ricin toxin, and cytosine deaminase (5-fluorocytosine).

The nucleotide sequence encoding the negative selectable marker is positioned outside of the two homology regions of the replacement vector. Given this positioning, cells will only integrate and stably express the negative selectable marker if integration occurs by random, non-homologous recombination; homologous recombination between the REDK gene and the two regions of homology in the targeting construct excludes the sequence encoding the negative selectable marker from integration. Thus, by imposing the negative condition, cells that have integrated the targeting vector by random, non-homologous recombination lose viability.

The above-described combination of positive and negative selectable markers is preferred in a targeting construct used to make the genetically-modified non-human mammals and mammal cells of the invention because a series of positive and negative selection steps can be designed to more efficiently select only those cells that have undergone vector integration by homologous recombination, and, therefore, have a potentially disrupted REDK gene. Further examples of positive-negative selection schemes, selectable markers, and targeting constructs are described, for example, in U.S. Pat. No. 5,464,764, WO 94/06908 (counterpart U.S. Pat. No. 5,859,312), and Valancius and Smithies (1991).

In order for a marker protein to be stably expressed upon vector integration, the targeting vector may be designed so that the marker coding sequence is operably linked to the endogenous REDK gene promoter upon vector integration. Expression of the marker is then driven by the REDK gene promoter in cells that normally express the REDK gene. Alternatively, each marker in the targeting construct of the vector may contain its own promoter that drives expression independent of the REDK gene promoter. This latter scheme has the advantage of allowing for expression of markers in cells that do not typically express the REDK gene (Smith and Berg (1984); Sedivy and Sharp (1989); Thomas and Capecchi (1987)).

Exogenous promoters that can be used to drive marker gene expression include cell-specific or stage-specific promoters, constitutive promoters, and inducible or regulatable promoters. Non-limiting examples of these promoters include the herpes simplex thymidine kinase promoter, cytomegalovirus (CMV) promoter/enhancer, SV40 promoters, PGK promoter, PMC1-neo, metallothionein promoter, adenovirus late promoter, vaccinia virus 7.5K promoter, avian beta globin promoter, histone promoters (e.g., mouse histone H3-614), beta actin promoter, neuron-specific enolase, muscle actin promoter, and the cauliflower mosaic virus 35S promoter (see generally, Sambrook et al. (1989), and Ausubel, F.M. et al. (2001); Stratagene, La Jolla, CA).

To confirm whether cells have integrated the vector sequence into the targeted REDK gene locus while making the genetically-modified non-human mammals and animal cells of the invention, primers or genomic probes that are specific for the desired vector integration event can be used in combination with PCR or Southern blot analysis to identify the presence of the desired vector integration into the REDK gene locus (Erlich et al. (1991); Zimmer and Gruss (1989); Mouellic et al. (1990); and Shesely, et al. (1991).

### 2. Gene Trapping

Another method available for inserting a foreign nucleic acid sequence into the REDK gene locus to disrupt the REDK gene, based on the present description, is gene trapping. This method takes advantage of the cellular machinery present in all mammalian cells that splices exons into mRNA to insert a gene trap vector coding sequence into a gene in a random fashion. Once inserted, the gene trap vector creates a mutation that may disrupt the trapped REDK gene. In contrast to homologous recombination, this system for mutagenesis creates largely random mutations. Thus, to obtain a genetically-modified cell that contains a disrupted REDK gene, cells containing this particular mutation must be identified and selected from a pool of cells that contain random mutations in a variety of genes.

Gene trapping systems and vectors have been described for use in genetically modifying murine cells and other cell types (see, for example, Allen et al. (1988); Bellen et al. (1989); Bier et al. (1989); Bonnerot et al. (1992); Brenner et al. (1989); Chang et al. (1993); Friedrich and Soriano (1993); Friedrich and Soriano (1991); Goff (1987); Gossler et al. (1989); Hope (1991); Kerr et al. (1989); Reddy et al. (1991); Reddy et al. (1992); Skames et al. (1992); von Melchner and Ruley (1989); and Yoshida et al. (1995).

Promoter trap vectors (or 5' vectors) contain, in 5' to 3' order, a splice acceptor sequence followed by an exon, which is typically characterized by a translation initiation codon and open reading frame and/or an internal ribosome entry site. In general, these promoter trap vectors do not contain promoters or operably linked splice donor sequences. Consequently, after integration into the cellular genome of the host cell, the promoter trap vector sequence intercepts the normal splicing of the upstream gene and acts as a terminal exon. Expression of the vector coding sequence is dependent upon the vector integrating into an intron of the disrupted gene in the proper reading frame. In such a case, the cellular splicing machinery splices exons from the trapped gene upstream of the vector coding sequence (e.g., WO 99/50426 and U.S. Pat. No. 6,080,576).

An alternative method for producing an effect similar to the above-described promoter trap vector is a vector that incorporates a nested set of stop codons present in, or otherwise engineered into, the region between the splice acceptor of the promoter trap vector and the translation initiation codon or polyadenylation sequence. The coding sequence can also be engineered to contain an independent ribosome entry site (IRES) so that the coding sequence will be expressed in a manner largely independent of the site of integration within the host cell genome. Typically, but not necessarily, an IRES is used in conjunction with a nested set of stop codons.

Another type of gene trapping scheme uses a 3' gene trap vector. This type of vector contains, in operative combination, a promoter region, which mediates expression of an adjoining coding sequence, the coding sequence, and a splice donor sequence that defines the 3' end of the coding sequence exon. After integration into a host cell genome, the transcript expressed by the vector promoter is spliced to a splice acceptor sequence from the trapped gene that is located downstream of the integrated gene trap vector sequence. Thus, the integration of the vector results in the expression of a fusion transcript comprising the coding sequence of the 3' gene trap cassette and any downstream cellular exons, including the terminal exon and its polyadenylation signal. When such vectors integrate into a gene, the cellular splicing machinery splices the vector coding sequence upstream of the 3' exons of the trapped gene. One advantage of such vectors is that the expression of the 3' gene trap vectors is driven by a promoter within the gene trap cassette and does not require integration into a gene that is normally expressed in the host cell (WO 99/50426 and U.S. Pat. No. 6,080,576). Examples of transcriptional promoters and enhancers that may be incorporated into the 3' gene trap vector include those discussed above with respect to targeting vectors.

The viral vector backbone used as the structural component for the promoter or 3' gene trap vector may be selected from a wide range of vectors that can be inserted into the genome of a target cell. Suitable backbone vectors include, but are not limited to, herpes simplex virus vectors, adenovirus vectors, adeno-associated virus vectors, retroviral vectors, lentiviral vectors, pseudorabies virus, alpha-herpes virus vectors, and the like. A thorough review of viral vectors, in particular, viral vectors suitable for modifying nonreplicating cells and how to use such vectors in conjunction with the expression of an exogenous polynucleotide sequence, can be found in Caplitt and Loewy (1995).

Preferably, retroviral vectors are used for gene trapping. These vectors can be used in conjunction with retroviral packaging cell lines such as, for example, those described in U.S. Patent No. 5,449,614. Where non-murine mammalian cells are used as target cells for genetic modification, amphotropic or pantropic packaging cell lines can be used to package suitable vectors (see, for example, Ory et al. (1996)). Representative retroviral vectors that can be adapted to create the presently described 3' gene trap vectors are described, for example, in U.S. Pat. No. 5,521,076.

The gene trapping vectors may contain one or more of the positive marker genes discussed above with respect to targeting vectors used for homologous recombination. Similar to their use in targeting vectors, these positive markers are used in gene trapping vectors to identify and select cells that have integrated the vector into the cell genome. The marker gene may be engineered to contain an IRES so that the marker will be expressed in a manner largely independent of the location in which the vector has integrated into the target cell genome.

Given that gene trap vectors will integrate into the genome of infected host cells in a fairly random manner, a genetically-modified cell having a disrupted REDK gene must be identified from a population of cells that have undergone random vector integration. Preferably, the genetic modifications in the population of cells are of sufficient randomness and frequency such that the population represents mutations in essentially every gene found in the cell's genome, making it likely that a cell with a disrupted REDK gene will be identified from the population (see WO 99/50426; WO 98/14614 and U.S. Pat. No. 6,080,576).

Individual mutant cell lines containing a disrupted REDK gene are identified in a population of mutated cells using, for example, reverse transcription and PCR to identify a mutation in a REDK gene sequence. This process can be streamlined by pooling clones. For example, to find an individual clone containing a disrupted REDK gene, RT-PCR is performed using one primer anchored in the gene trap vector and the other primer located in the REDK gene sequence. A positive RT-PCR result indicates that the vector sequence is encoded in the REDK gene transcript, indicating that the REDK gene has been disrupted by a gene trap integration event (see, for example, WO 98/14614, U.S. Pat. No. 6,080,576).

### 3. Temporal, Spatial, and Inducible REDK Gene Disruptions

In certain embodiments of the present invention, a functional disruption of the endogenous REDK gene occurs at specific developmental or cell cycle stages (temporal disruption) or in specific cell types (spatial disruption). In other embodiments, the REDK gene disruption is inducible when certain conditions are present. A recombinase excision system, such as a Cre-Lox system, may be used to activate or inactivate the REDK gene at a specific developmental stage, in a particular tissue or cell type, or under particular environmental conditions. Generally, methods utilizing Cre-Lox technology are carried out as described by Torres and Kuhn (1997). Methodology similar to that described for the Cre-Lox system can also be employed utilizing the FLP-FRT system. Further guidance regarding the use of recombinase excision systems for conditionally disrupting genes by homologous recombination or viral insertion is provided, for example, in U.S. Pat. No. 5,626,159, U.S. Pat. No. 5,527,695, U.S. Pat. No. 5,434,066, WO 98/29533, U.S. Pat. No. 6,228,639, Orban et al. (1992); O'Gorman et al. (1991); Sauer et al. (1989); Barinaga (1994); and Akagi et al. (1997). More than one recombinase system can be used to genetically modify a non-human mammal or mammal cell of the present invention.

When using homologous recombination to disrupt the REDK gene in a temporal, spatial, or inducible fashion, using a recombinase system such as the Cre-Lox system, a portion of the REDK gene coding region is replaced by a targeting construct comprising the REDK gene coding region flanked by loxP sites. Non-human mammals and mammal cells carrying this genetic modification contain a functional, loxP-flanked REDK gene. The temporal, spatial, or inducible aspect of the REDK gene disruption is caused by the expression pattern of an additional transgene, a Cre recombinase transgene, that is expressed in the non-human mammal or mammal cell under the control of the desired spatially-regulated, temporally-regulated, or inducible promoter, respectively. A Cre recombinase targets the loxP sites for recombination. Therefore, when Cre expression is activated, the LoxP sites undergo recombination to excise the sandwiched REDK gene coding sequence, resulting in a functional disruption of the REDK gene (see, for example, Rajewski et al. (1996); St.-Onge et al. (1996); Agah et al. (1997); Brocard et al. (1997); Feil et al. (1996); and Kühn et al. (1995)).

A cell containing both a Cre recombinase transgene and loxP-flanked REDK gene can be generated through standard transgenic techniques or, in the case of genetically-modified, non-human mammals, by crossing genetically-modified, non-human mammals wherein one parent contains a loxP flanked REDK gene and the other contains a Cre recombinase transgene under the control of the desired promoter. Further guidance regarding the use of recombinase systems and specific promoters to temporally, spatially, or conditionally disrupt the REDK gene is found, for example, in Sauer (1993), Gu et al. (1994), Araki et al. (1997), Dymecki (1996), and Meyers et al. (1998).

An inducible disruption of the REDK gene can also be achieved by using a tetracycline responsive binary system (Gossen and Bujard (1992)). This system involves genetically modifying a cell to introduce a Tet promoter into the endogenous REDK gene regulatory element and a transgene expressing a tetracycline-controllable repressor (TetR). In such a cell, the administration of tetracycline activates the TetR which, in turn, inhibits REDK gene expression and, therefore, disrupts the REDK gene (see, for example, St.-Onge et al. (1996) and U.S. Patent No. 5,922,927).

The above-described systems for temporal, spatial, and inducible disruptions of the REDK gene can also be adopted when using gene trapping as the method of genetic modification, for example, as described, in WO 98/29533 and U.S. Pat. No. 6,288,639, for creating the genetically-modified non-human mammals and mammal cells of the invention.

### Preparation of Genetically-Modified Mammal cells

The above-described methods for genetic modification can be used to disrupt a REDK gene in virtually any type of somatic or stem cell derived from an animal, preferably a mammal, to create the genetically-modified animal cells of the invention. Genetically-modified animal cells of the invention include, but are not limited to, mammalian cells, including human cells, and avian cells. These cells may be derived from genetically engineering any mammal cell line, such as culture-adapted, tumorigenic, or transformed cell lines, or they may be isolated from a genetically-modified, non-human mammal carrying the desired REDK genetic modification.

The cells may be heterozygous or homozygous for the disrupted REDK gene. To obtain cells that are homozygous for the REDK gene disruption (-/-), direct, sequential targeting of both alleles can be performed. This process can be facilitated by recycling a positive selectable marker. According to this scheme the nucleotide sequence encoding the positive selectable marker is removed following the disruption of one allele using the Cre-Lox P system. Thus, the same vector can be used in a subsequent round of targeting to disrupt the second REDK gene allele (Abuin and Bradley (1996); Sedivy et al. (1999); Cruz et al. (1991); Mortensen et al. (1991); te Riele et al. (1990)).

An alternative strategy for obtaining ES cells that are REDK (-/-) is a method disclosed in Mortensen, et al. (1992). In this method, REDK (+/-) targeted clones that express a selectable drug resistance marker are selected against a very high drug concentration; this selection favors cells that express two copies of the sequence encoding the drug resistance marker and are, therefore, homozygous for the REDK gene disruption. In addition, genetically-modified mammal cells can be obtained from genetically-modified REDK (-/-) non-human mammals that are created by mating non-human mammals that are REDK (+/-) in germline cells, as further discussed below.

Following the genetic modification of the desired cell or cell line, the REDK gene locus can be confirmed as the site of modification by PCR analysis according to standard PCR or Southern blotting methods known in the art (see, for example, U.S. Pat. No. 4,683,202; and Erlich et al. (1991)). Further verification of the functional disruption of the REDK gene may also be made if REDK gene messenger RNA (mRNA) levels and/or REDK polypeptide levels are reduced in cells that normally express the REDK gene. Measures of REDK gene mRNA levels may be obtained by using reverse transcriptase mediated polymerase chain reaction (RT-PCR), Northern blot analysis, or in situ hybridization. The quantification of REDK polypeptide levels produced by the cells can be made, for example, by standard immunoassay methods known in the art. Such immunoassays include, but are not limited to, competitive and non-competitive assay systems using techniques such as RIAs (radioimmunoassays), ELISAs (enzyme-linked immunosorbent assays), "sandwich" immunoassays, immunoradiometric assays, gel diffusion precipitin reactions, immunodiffusion assays, in situ immunoassays (using, for example, colloidal gold, enzymatic, or radioisotope labels), Western blots, 2-dimensional gel analysis, precipitation reactions, immunofluorescence assays, protein A assays, and immunoelectrophoresis assays.

Preferred genetically-modified mammal cells of the invention are ES cells and ES-like cells. These cells are derived from the preimplantation embryos and blastocysts of various species, such as mice (see, for example, Evans et al. (1981); Martin (1981)), pigs and sheep (see, for example, Notanianni, et al. (1991); Campbell et al. (1996)) and primates, including humans (see, for example, U.S. Patent No. 5,843,780 and Thomson et al. (1995)).

These types of cells are pluripotent, that is, under proper conditions, they differentiate into a wide variety of cell types derived from all three embryonic germ layers: ectoderm, mesoderm and endoderm. Depending upon the culture conditions, a sample of ES cells can be cultured indefinitely as stem cells, allowed to differentiate into a wide variety of different cell types within a single sample, or directed to differentiate into a specific cell type, such as macrophage-like cells, neuronal cells, cardiomyocytes, chondrocytes, adipocytes, smooth muscle cells, endothelial cells, skeletal muscle cells, keratinocytes, and hematopoietic cells, such as eosinophils, mast cells, erythroid progenitor cells, or megakaryocytes. Directed differentiation is accomplished by including specific growth factors or matrix components in the culture conditions, as further described, for example, in Keller et al. (1995), Li et al. (1998), Klug et al. (1996), Lieschke et al. (1995), Yamane et al. (1997), and Hirashima et al. (1999).

The particular ES cell line that is used for genetic modification is not critical. For example, for those embodiments of this invention wherein murine ES cell lines are used, such cells may include AB-1 (McMahon and Bradley (1990)), E14 (Hooper, et al. (1987)), D3 (Doetschman, et al. (1985)), CCE (Robertson, et al. (1986)), RW4 (Genome Systems, St. Louis, MO), and DBA/1IacJ (Roach et al. (1995)).

### Preparation of Genetically-Modified, Non-human Mammals

The genetically-modified mammal cells of this invention may be used to prepare genetically-modified, non-human mammals of this invention. In one embodiment, genetically-modified ES cells of this invention may be used to generate genetically-modified non-human mammals, according to published procedures (see, for example, Robertson (1987), pp. 71-112; Zjilstra et al.(1989); and Schwartzberg, et al. (1989)).

In a preferred embodiment, said ES cells are murine ES cells and said genetically-modified non-human mammals, are mice. For example, in the preparation of such genetically-modified mice, murine ES cells containing the desired functional disruption of the REDK gene are used. The murine ES cells to be used are first confirmed to contain the desired functional disruption of the REDK gene, as described above.

The REDK disrupted ES cells may then be used to generate chimeric mice according to methods known in the art *(see, for example,* Capecchi (1989)). For example, the REDK disrupted ES cells may be injected into suitable blastocyst hosts. The particular mouse blastocysts employed in the practice of the present invention are not critical. Examples of such blastocysts will be known to those with skill in the art in light of the present description, and include blastocysts derived from C57BL6 mice, C57BL6 Albino mice, Swiss outbred mice, CFLP mice, and MFI mice. Alternatively ES cells may be sandwiched between tetraploid embryos in aggregation wells (see, for example, Nagy et al. (1993) Proc. Natl. Acad. Sci. USA 90:8424-8428).

The blastocysts or embryos containing the genetically-modified ES cells are then implanted in pseudopregnant female mice and allowed to develop in utero (see, for example, Hogan, et al. (1988); and E.J. Robertson (1987)). The offspring bom to the foster mothers may be screened to identify those that are chimeric for the REDK gene disruption. Generally, such offspring contain some cells that are derived from the genetically-modified donor ES cell as well as other cells derived from the original blastocyst. In such circumstances, offspring may be screened initially for mosaic coat color, where a coat color selection strategy has been employed, to distinguish cells derived from the donor ES cell from the other cells of the blastocyst. Alternatively, DNA from tail tissue of the offspring can be used to identify mice containing the genetically-modified cells.

The mating of chimeric mice that contain the REDK gene disruption in germ line cells produces progeny that possess the REDK gene disruption in all germ line cells and somatic cells. Mice that are heterozygous for the REDK gene disruption can then be crossed to produce homozygotes (see, for example, U.S. Pat. No. 5,557,032, and U.S. Pat. No. 5,532,158).

An alternative to the above-described ES cell technology for transferring a genetic modification from a cell to a whole animal is to use nuclear transfer. This method can be employed to make other genetically-modified, non-human mammals besides mice, for example, sheep (see, for example, McCreath, et al. (2000); Campbell, et al. (1996); and Schnieke, et al. (1997)) and calves (Cibelli et al. (1998)). Briefly, somatic cells (e.g., fibroblasts) or pluripotent stem cells (e.g., ES-like cells) are selected as nuclear donors and are genetically-modified to contain a functional disruption of the REDK gene. When inserting a DNA vector into a somatic cell to mutate the REDK gene, it is preferred that a promoterless marker be used in the vector such that vector integration into the REDK gene results in expression of the marker under the control of the REDK gene promoter (Sedivy, et al. (1999); McCreath, et al. (2000)). Nuclei from donor cells which have the appropriate REDK gene disruption are then transferred to fertilized or parthenogenetic oocytes that are enucleated (Campbell, et al. (1996); Wilmut, et al. (1997)). Embryos are reconstructed, cultured to develop into the morula/blastocyst stage, and transferred into foster mothers for full term in utero development.

The present invention also encompasses the progeny of the genetically-modified, non-human mammals and genetically-modified mammal cells. While the progeny are heterozygous or homozygous for the genetic modification that disrupts the REDK gene, they may not be genetically identical to the parent non-human mammals and mammal cells due to mutations or environmental influences, besides that of the original genetic disruption of the REDK gene, that may occur in succeeding generations.

The cells from a non-human genetically-modified animal can be isolated from tissue or organs using techniques known to those of skill in the art. In one embodiment, the genetically-modified cells of the invention are immortalized. In accordance with this embodiment, cells can be immortalized by genetically engineering the telomerase gene, an oncogene, e.g., mos or v-src, or an apoptosis-inhibiting gene, e.g., bcl-2, into the cells. Alternatively, cells can be immortalized by fusion with a hybridization partner utilizing techniques known to one of skill in the art.

The genetically-modified non-human mammals and animal cells of the invention containing a disrupted endogenous REDK gene can be further modified to express the human REDK sequence (referred to herein as "humanized"). A preferred method for humanizing cells involves replacing the endogenous REDK sequence with nucleic acid sequence encoding the human REDK sequence (see, for example, Jakobsson, et al. (1999)) by homologous recombination. The vectors are similar to those traditionally used as targeting vectors with respect to the 5' and 3' homology arms and positive/negative selection schemes. However, the vectors also include sequence that, after recombination, either substitutes the human REDK coding sequence for the endogenous sequence, or effects base pair changes, exon substitutions, or codon substitutions that modify the endogenous sequence to encode the human REDK. Once homologous recombinants have been identified, it is possible to excise any selection-based sequences (e.g., neo) by using Cre- or Flp-mediated site directed recombination (Dymecki (1996)).

When substituting the human REDK sequence for the endogenous sequence, it is preferred that these changes are introduced directly downstream of the endogenous translation start site. This positioning preserves the endogenous temporal and spatial expression patterns of the REDK gene. The human sequence can be the full length human cDNA sequence with a polyA tail attached at the 3' end for proper processing or the whole genomic sequence (see, for example, Shiao et al. (1999)). Further guidance regarding these methods of genetically modifying cells and non-human mammals to replace expression of an endogenous gene with its human counterpart is provided, for example, in Sullivan, et al. (1997), Reaume, et al. (1996), and U.S. Pat. No. 5,777,194.

Another method known in the art for creating such "humanized" organisms is a two-step process involving the disruption of the endogenous gene followed by the introduction of a transgene encoding the human sequence by pronuclear microinjection into the knock-out embryos.

### Uses of Genetically-Modified Non-human Mammals and Animal Cells

The function and therapeutic relevance of the REDK gene may be further elucidated by investigating phenotype characteristics of REDK (-/-) non-human mammals and animals cells of the invention. For example, the genetically-modified REDK (-/-) non-human mammals and mammal cells can be used to determine whether the REDK plays a role in causing or preventing symptoms or phenotypes to develop in certain models of disease, e.g., anemia. If a symptom or phenotype is different in a REDK (-/-) non-human mammal or animal cell as compared to a wild type (REDK +/+) or REDK (+/-) non-human mammal or animal cell, then the REDK polypeptide plays a role in regulating functions associated with the symptom or phenotype.

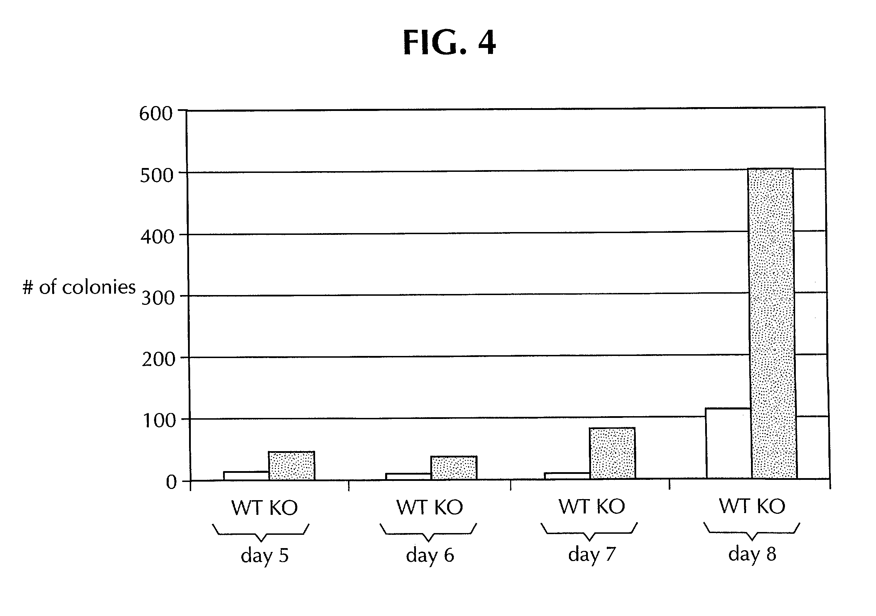

REDK function may be investigated by comparing REDK disrupted ES cells or REDK (-/-) mice to wild type ES cells or mice with respect to a biological characteristic, such as a morphologic, a histologic, a metabolic, developmental, or behavioral characteristic, as applicable. An exemplary biological characteristic of REDK (-/-) ES cells that may be used to study REDK function is the effect of REDK disruption on erythroid differentiation. For example, REDK (-/-) ES cells of the invention and wild-type cells may be used to prepare embryoid bodies by the removing leukemia inhibitory factor (LIF) from the culture medium. The cells of the embryoid bodies are then cultured in the presence of erythropoietin, and the level of erythroid differentiation may be compared in REDK (-/-) and wild-type cells by assessing the formation of red-colored erythroid colonies. As illustrated in Figure 4, the level of erythroid differentiation is substantially enhanced in REDK (-/-) ES cells as compared to wild-type cells.

In addition, under circumstances in which an agent has been identified as a REDK agonist or antagonist (e.g., the agent significantly modifies one or more of the REDK polypeptide activities when the agent is administered to a REDK (+/+) or REDK (+/-) non-human mammal or animal cell), the genetically-modified REDK (-/-) non-human mammals and animal cells of the invention are useful to characterize any other effects caused by the agent besides those known to result from the antagonism of REDK (i.e., the non-human mammals and animal cells can be used as negative controls). For example, if the administration of the agent causes an effect in a REDK (+/+) non-human mammal or animal cell that is not known to be associated with REDK polypeptide activity, then one can determine whether the agent exerts this effect solely or primarily through modulation of REDK by administering the agent to a corresponding REDK (-/-) non-human mammal or animal cell. If this effect is absent, or is significantly reduced, in the REDK (-/-) non-human mammal or animal cell, then the effect is mediated, at least in part, by REDK. However, if the REDK (-/-) non-human mammal or animal cell exhibits the effect to a degree comparable to the REDK (+/+) or REDK (+/-) non-human mammal or animal cell, then the effect is mediated by a pathway that does not involve REDK signaling.

Furthermore, if an agent is suspected of possibly exerting an effect predominantly via a REDK pathway, then the REDK (-/-) non-human mammals and mammal cells are useful as negative controls to test this hypothesis. If the agent is indeed acting through REDK, then the REDK (-/-) non-human mammals and mammal cells, upon administration of the agent, should not demonstrate the same effect observed in the REDK (+/+) non-human mammals or mammal cells.

The genetically-modified non-human mammals and mammal cells of the invention can also be used to identify genes whose expression is differentially regulated in REDK (+/-) or REDK (-/-) non-human mammals or mammal cells relative to their respective wild-type control. Techniques known to those of skill in the art can be used to identify such genes based upon the present description. For example, microarrays can be used to identify genes whose expression is differentially regulated in REDK (+/-) or REDK (-/-) mice to compensate for a deficiency in REDK expression. Microarrays known to those of skill in the art may be prepared, used, and the results analyzed using methods known in the art (see, for example, Aigner, et al. (2001); U.S. Patent No. 5,965,352; Schena, et al. (1995-A); DeRisi, et al. (1996); Shalon, et al. (1996); and Schena, et al. (1995-B); U.S. Patent No. 5,474,796; Schena, M., et al. (1996); WO 95/251116; WO 95/35505; Heller, R.A. et al. (1997); and U.S. Patent No. 5,605,662).

A chemical coupling procedure and an ink jet device may be used to synthesize array elements on the surface of a substrate (see, e.g., Baldeschweiler, supra). An array analogous to a dot or slot blot may also be used to arrange and link elements to the surface of a substrate using thermal, UV, chemical, or mechanical bonding procedures. A typical array may be produced by hand or using available methods and machines and contain any appropriate number of elements. After hybridization, nonhybridized probes are removed and a scanner used to determine the levels and patterns of fluorescence. The degree of complementarity and the relative abundance of each probe which hybridizes to an element on the microarray may be assessed through analysis of the scanned images.

Full-length cDNAs, expressed sequence tags (ESTs), or fragments thereof may comprise the elements of a microarray. Fragments suitable for hybridization may be selected using software well known in the art such as LASERGENE software (DNASTAR). Full-length cDNAs, ESTs, or fragments thereof corresponding to one of the nucleotide sequences of the present invention, or selected at random from a cDNA library relevant to the present invention, are arranged on an appropriate substrate, e.g., a glass slide. The cDNA is fixed to the slide using, e.g., ultra-violet cross-linking followed by thermal and chemical treatments and subsequent drying (see, e.g., Schena, M. et al. (1995-A); Shalon, et al. (1996)). Fluorescent probes are prepared and used for hybridization to the elements on the substrate. The substrate is analyzed by procedures well known in the art, for example, by scanning and analyzing images of a microarray.

In addition, the genetically-modified non-human mammals and mammal cells of the present invention can be used to identify proteins whose expression or postranslational modification is altered in REDK (+/-) or REDK (-/-) non-human mammals or mammal cells relative to their respective wild-type control. Techniques known to those of skill in the art can be used to identify such proteins based upon the present description. For example, proteomic assays may be used to identify proteins whose expression profile or postranslational modification is altered in REDK (+/-) or REDK (-/-) mice to compensate for a deficiency in REDK expression. Proteomic assays are known to those of skill in the art (see, for example, Conrads et al. (2002); Dongre, et al. (2001); Van Eyk (2001); Cole, et al. (2000); Araki, et al. (2000)).

### EXAMPLES

### Example 1

### Preparation of REDK Targeting Vector

A 1 Kb murine REDK genomic fragment, containing base pairs 1121-2092 of the murine REDK cDNA sequence (Genbank BC006704), was used as a probe to identify genomic clones from a 129/SvJ genomic λ-phage library (Stratagene). Two positive λ-clones where isolated containing overlapping 12 Kb and 17 Kb fragments.

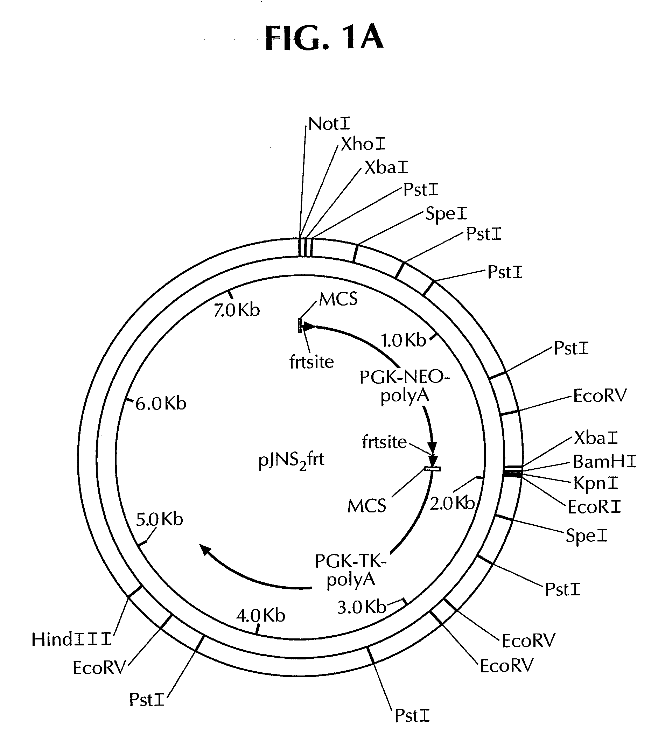

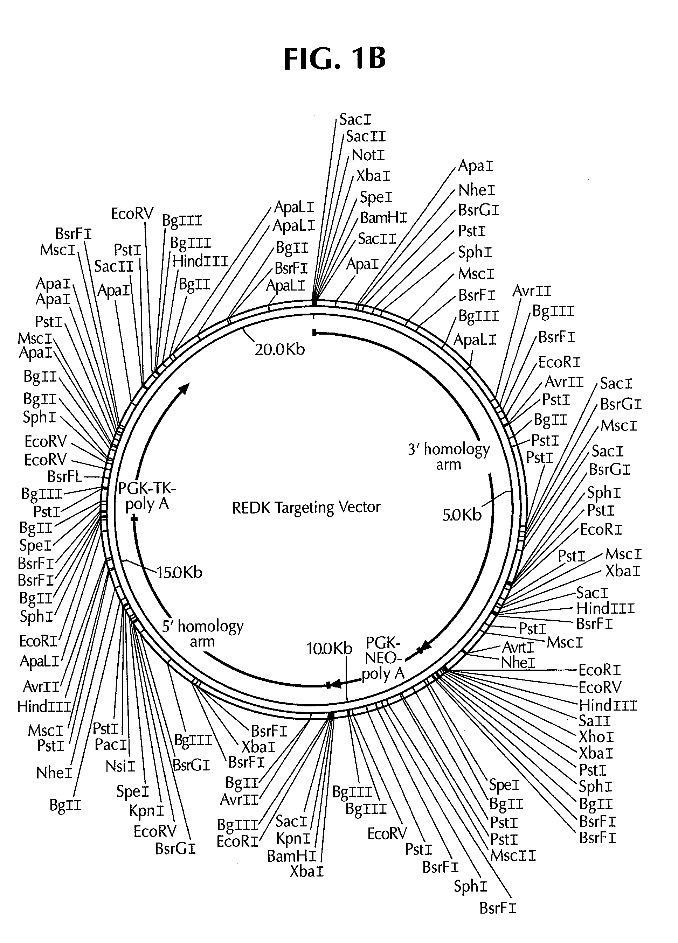

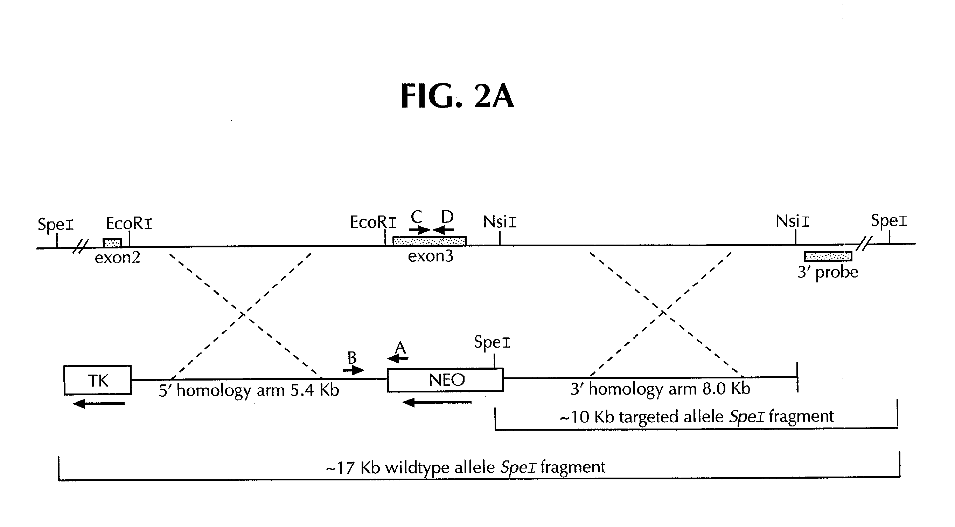

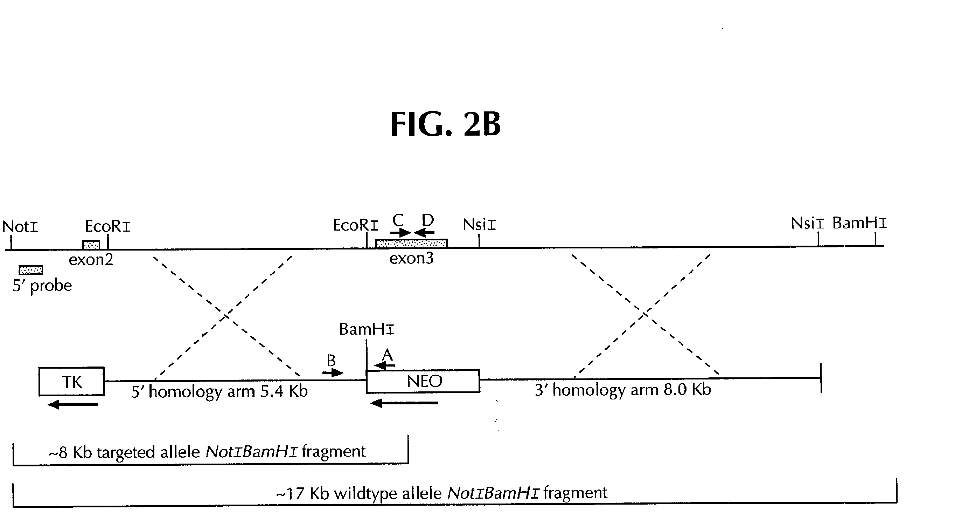

The clone containing the 12 Kb fragment was cut with EcoRI to make a 5.4 Kb fragment to be used as the 5' homology arm of the targeting vector. The 5.4 Kb fragment was then inserted into the EcoRI site of the backbone vector pJNS₂frt (Figure 1). The clone containing the 17 Kb fragment was cut using Nsil to make an 8 Kb fragment. The 8 Kb fragment was inserted into the Pst site of pBluescriptIISK⁺ (Stratagene). The pBluescriptIISK⁺ clone was restricted with NotI/XhoI to make an 8 Kb fragment, which was then inserted as the 3' homology arm into the NotI/XhoI site of the pJNS₂frt backbone vector already containing the 5' homology arm 5.4 Kb fragment. As indicated in Figures 2A and 2B, the selectable markers are in opposite orientation to the homology arms.

The resulting targeting vector was used to prepare the REDK disrupted ES cells, as described in Example 2 below, by replacing approximately 3 Kb of genomic locus, corresponding to amino acids 64-586 of the murine REDK peptide sequence (GenBank accession BC006704) with the neomycin gene, PGK-NEO. This creates a deletion from isoleucine 64 to serine 586 within the native protein.

### Example 2

### Preparation of REDK Disrupted ES Cells

*Procedure A - Culture Conditions.* Pluripotent RW4 ES cells derived from 129svJ inbred mouse strain were maintained in culture on a mitomyocin C treated primary embryonic fibroblast (PEF) feeder layer in stem cell medium (SCML) containing Dulbecco's Modified Eagle's Medium (DMEM) (#10829-018, Invitrogen Life Technologies, Inc., Carlsbad, CA) supplemented with 15% ES cell qualified fetal calf serum (#10439-024, Invitrogen Life Technologies), 0.1 mM 2-mercaptoethanol (#M-7522, Sigma-Aldrich, St. Louis, MO), 0.2 mM L-glutamine (#25030-081, Invitrogen Life Technologies), 0.1 mM Modified Eagle's Medium (MEM) non-essential amino acids (#11140-050, Invitrogen Life Technologies), 1000 u/ml recombinant murine leukemia inhibitory factor (LIF) (ESGRO®, # ESG-1107, Chemicon International, Inc., Temecula, CA) and 50 µg/ml gentamicin (#15710-064, Invitrogen Life Technologies).

*Procedure B - Preparation of REDK (+*/*-) ES cells*. The REDK targeting vector of Example 1 (25 µg) was linearized and electroporated in 1x10⁷ RW4 ES cells in 400 µl SCML using a BTX Electro Cell Manipulator 600 (BTX, Inc., San Diego, CA) at a voltage of 260 V, a capacitance of 50 µF and a resistance of 360 ohms. Following electroporation, the cells were plated in SCML in four 100 mm tissue culture dishes on mitomycin C treated PEFs. Twenty-four hours after electroporation, positive/negative selection was initiated by adding 200 µg/ml G418 (#10131-035, Invitrogen Life Technologies) and 2 µM gancyclovir to the SCML. After 7-9 days of selection, G418 resistant colonies were picked with a drawn micropipette into individual wells of a 24-well tissue culture dish and expanded into clonal ES cell lines.

*Procedure C - Preparation of REDK (-*/*-) ES Cells*. REDK (+/-) ES cell of Procedure B of this Example were thawed and maintained on PEFs in 200µg/ml G418/SCML for two days. The concentration of G418 (#11811-031, Invitrogen Life Technologies) was then increased to 2.5mg/ml. After 7-10 days, the surviving ES cell colonies were dissociated and 2-5 x 10⁵ cells/ml were plated onto new PEFs in 2.5 mg/ml G418/SCML. After 4-7 days, resistant colonies were picked with a drawn micropipette and transferred into individual wells of a 24-well tissue culture dish and expanded into clonal ES cell lines.

*Procedure D - Confirmation of Gene Targeting.*
Gene targeting by homologous recombination was determined in surviving ES cell clones by Southern analysis of *Spe*I digested ES cell DNA using a 1.0 Kb length probe from a region downstream of the 8.0 Kb homology arm. The endogenous wild-type allele yielded a band hybridizing at approximately 17 Kb, whereas the targeted (disrupted) allele gave a predicted restriction fragment length polymorphism (RFLP) of 10 Kb due to the introduction of a novel *Spel* site from the REDK targeting vector. Gene targeting by homologous recombination was confirmed on the 5' side by probing *BamH*I/*Not*I double digested ES cell DNA with a 650 bp length probe from a region upstream of the 6.0 Kb homology arm. This digest also resulted in a 17 Kb band for the wild-type allele and a 10 Kb band in the disrupted allele. Southern analysis using a NEO probe confirmed that none of the homologously targeted ES cell clones contained multiple insertions. Karyotyping analysis confirmed that no chromosome abnormalities occurred.

### Example 3

### Preparation of REDK Disrupted Mice

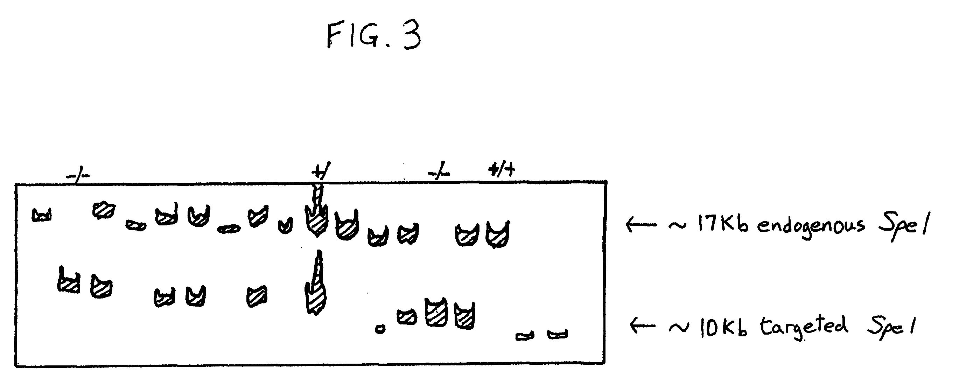

REDK (+/-) ES cells, of Example 2, were microinjected into 2.5 day old C57BL/6 blastocysts prepared according to Hogan, et al. (1988) from super-ovulated female mice (Charles River Laboratories, Inc., Wilmington, MA). The resulting chimeric mice were backcrossed to C57BL/6 mates to identify germline transmission. REDK (-/-) mice were derived from crosses of REDK (+/-) males and females. All animal work was performed according to protocols approved by the Pfizer Institutional Animal Care and Use Committee. Genotyping of second generation (F2) REDK mice was accomplished using the Southern analysis according to Example 2, above, as well as by a combination of two PCR reactions, one specific for the targeting vector to amplify a 500 bp fragment (junction PCR: forward primer 5'-GCCAGCTCATTCCTCCACTCA-3' (primer A) (SEQ ID NO: 1), reverse primer 5'-ATTTCTTTGAGACAGAGACTGC-3' (primer B) (SEQ ID NO: 2)) and a second PCR reaction specific for the disrupted region to amplify a 700 bp fragment (disrupted region PCR: forward primer 5'-AGGTCATCGACTTTGGCTCC-3' (primer C) (SEQ ID NO: 3), reverse primer 5'-CACTAGCTAATCAGCTTTGGC-3' (primer D) (SEQ ID NO: 4)). Cycling conditions for both reactions were 30 cycles of 94°C for 30 seconds, 65°C for 30 seconds and 72°C for 1 minute using standard PCR reaction protocols. The results are shown in Figure 3. The REDK (-/-) mice were viable and displayed no overt phenotype upon gross observation.

### Example 4

### Phenotype Comparison of REDK Disrupted and Wild-Type Mammalian Cells - Differentiation into Erythroid Cells

Wild-type RW4 ES cells derived from 129svJ and REDK (-/-) ES cells prepared according to Example 2 were cultured as described in Procedure A of Example 2. The wild-type and REDK (-/-) ES were removed from their respective PEF feeders and grown on tissue culture dishes for two days in medium of Iscove's Modified Dulbecco's Medium (IMDM) (#31980-030, Invitrogen Life Technologies) supplemented with 15% ES cell qualified fetal calf serum, 0.1 mM 2-mercaptoethanol (#M-7522, Sigma-Aldrich), 0.2 mM L-glutamine (#25030-081, Invitrogen Life Technologies), 0.1 mM MEM non-essential amino acids (# 11140-050, Invitrogen Life Technologies), 1000 u/ml recombinant murine LIF (ESGRO®, # ESG-1107, Chemicon International) and 50 µg/ml gentamicin (#15710-064, Invitrogen Life Technologies). The cells were then dissociated and grown in suspension culture in IMDM supplemented with 15% ES cell qualified fetal calf serum, 2 mM L-Glutamine, 300 µg Transferrin (#13008-016, Invitrogen Life Technologies), 50 µg/ml L-Ascorbic Acid (#A-4403, Sigma-Aldrich), 5% PFHM-II (#12040-093, Invitrogen Life Technologies), 4 x 10⁻⁴ M Monothioglycerol (MTG) and 50 µg/ml gentamicin (#15710-064, Invitrogen Life Technologies). The cells were grown in suspension for six days (starting on day 0) to form cell aggregated embryoid bodies (EBs). The EBs were dissociated on days 5, 6, 7 and 8 with 0.05% trypsin EDTA and plated 1 x 10⁵ cells/35mm dish in medium of IMDM and 1% methylcellulose (StemCell Technologies, British Columbia, Canada) supplemented with 10% ES cell qualified fetal calf serum, 5% protein-free hybridoma medium (PFHM-II) (#12040-093, Invitrogen Life Technologies), 2 mM L-Glutamine, 5 u/ml Epogen® (Amgen, Inc., Thousand Oaks, CA), 100 ng/ml Stem Cell Factor (StemCell Technologies) and 50 µg/ml gentamycin (#15710-064, Invitrogen Life Technologies). Red-colored erythroid colonies were counted on days 12-15 of differentiation. Figure 4 compares erythroid cell differentiation in wild-type and REDK (-/-) ES cells.

### REFERENCES

Abuin and Bradley (1996) Mol. Cell. Biol. 16:1851-56.
Agah et al. (1997) J. Clin. Invest. 100:169-79.
Aigner, et al. (2001) Arthritis and Rheumatism 44:2777-89.
Allen et al. (1988) Nature 333:852-55.
Akagi et al. (1997) Nucleic Acids Res. 25:1766-73.
Araki et al. (1997) J. Biochem. 122:977-82.
Araki, et al. (2000) Electrophoresis 21:180-1889.
Ausubel, F.M. et al. (2001) Current Protocols in Molecular Biology, John Wiley & Sons, New York, NY.
Barinaga (1994) Science 265:26-28.
Baum et al. (1994) Biotechniques 17:1058-62.
Bellen et al. (1989) Genes Dev. 3:1288-1300.
Bier et al. (1989) Genes Dev. 3:1273-1287.
Biewenga et al. (1997) J. Neuroscience Methods 71:67-75.
Bolivar et al. (1977) Gene 2:95.
Bollag et al. (1989) Annu. Rev. Genet. 23:199-225.
Bonnerot et al. (1992) J. Virol. 66:4982-91.
Bradley et al. (1992) Biotechnol. 10:534.
Brenner et al. (1989) Proc. Nat. Acad. Sci. USA 86:5517-21.
Brocard et al. (1997) Proc. Natl. Acad. Sci. USA 94:14559-63.
Campbell, et al. (1996) Nature 380:64-68.
Capecchi (1989) Science 244:1288-92.
Capecchi (1989) Trends Genet. 5:70.
Caplitt and Loewy (1995) Viral Vectors: Gene Therapy and Neuroscience Applications, Academic Press, San Diego.
Chang et al. (1993) Virology 193:737-47.
Chu et al. (1987) Nucleic Acids Res. 15:1311-26.
Cibelli, et al. (1998) Science 280:1256-58.
Cole, et al. (2000) Electrophoresis 21:1772-1781.
Coligan, John E., et al. (1994) Current Protocols in Immunology, John Wiley & Sons, New York, NY.
Coligan, John E., et al. (2002) Current Protocols in Protein Protein Science, John Wiley & Sons, New York, NY.
Conrads et al. (2002) Biochem. Biophys. Res. Commun. 290:896-890.
Cruz et al. (1991) Proc. Natl. Acad. Sci. (USA) 88:7170-74.
Davis et al. (1986) Basic Methods in Molecular Biology, Elsevir Sciences Publishing, Inc., New York, NY.
Deng and Capecchi (1992) Mol. Cell. Biol. 12:3365-3371.
DeRisi, et al. (1996) Nature Genetics 14:457-460.
Doetschman, et al. (1985) J. Embryol. Exp. Morph. 87:27-45.
Dongre, et al. (2001) Bioploymers 60:206-211.
Dracopoli, et al. (1994) Current Protocols in Human Genetics, John Wiley & Sons, New York, NY.
Dymecki (1996) Proc. Natl. Acad. Sci. 93:6191-96.
Efrat et al. (1994) Proc. Natl. Acad. Sci. USA 91:2051-55.
Evans et al. (1981) Nature 129:154-156.
Erlich et al. (1991) Science 252:1643-51.
Feil et al. (1996) Proc. Natl. Acad. Sci. USA 93:10887-90.
Friedrich and Soriano (1991) Genes Dev. 5:1513-23.
Friedrich and Soriano (1993) Methods Enzymol. 225:681-701.
Goff (1987) Methods Enzymol. 152:469-81.
Gossen and Bujard (1992) Proc. Natl. Acad. Sci. USA 89:5547-51.
Gossler et al. (1989) Science 244:463-65.
Gu et al. (1994) Science 265:103-06.
Hames et al. (1985) Nucleic Acid Hybridization, IL Press.
Heller, R.A. et al. (1997) Proc. Natl. Acad. Sci. USA 94:2150-2155.
Hirashima et al. (1999) Blood 93:1253-63.
Hogan, et al. (1988) Manipulating the Mouse Embryo: A Laboratory Manual, Cold Spring Harbor Laboratory Press, Cold Spring Harbor, NY.
Hooper, et al. (1987) Nature 326:292-95.
Hope (1991) Develop. 113:399-408.
Jakobsson, et al. (1999) Proc. Natl. Acad. Sci. USA 96:7220-25.
Keller et al. (1995) Curr. Opin. Cell Biol. 7:862-69.
Kentrup, Heiner, et al. (1996) J. Biol. Chem. 271, 3488-3495.
Kerr et al. (1989) Cold Spring Harb. Symp. Quant. Biol. 2:767-776.
Klug et al. (1996) J. Clin. Invest. 98:216-24.
Kühn et al. (1995) Science 269:1427-29.
Larsson et al. (1994) Nucleic Acids Research 22:2242-48.
Li, et al. (1998) Curr. Biol. 8:971.
Lieschke et al. (1995) Exp. Hematol. 23:328-34.
Linney et al. (1999) Dev. Biol. (Orlando) 213:207-16.
Lo (1983) Mol. Cell. Biol. 3:1803-14.
Lord, Kenneth A., et al. (2000) Blood 95(9), 2838-2846.
Luyckx et al. (1999) Proc. Natl. Acad. Sci. 96:12174-79.
Mansour (1990) GATA 7:219-227.
Mansour et al. (1988) Nature 336:348-52.
Mansour et al. (1990) Proc. Natl. Acad. Sci. USA 87:7688-7692.
Mattila, et al. (1991) Nucleic Acids Res. 19:4967.
Martin (1981) Proc. Natl. Acad. Sci., USA, 78:7634-7638.
Meyers et al. (1998) Nature Genetics 18:136-41.
McCreath, et al. (2000) Nature 29:1066-69.
McMahon and Bradley (1990) Cell 62:1073-85.
Mortensen et al. (1991) Proc. Natl. Acad. Sci. (USA) 88:7036-40.
Mortensen, et al. (1992) Mol. Cell. Biol. 12:2391-95
Mouellic et al. (1990) Proc. Natl. Acad. Sci. (USA) 87:4712.
Nagy et al. (1993) Proc. Natl. Acad. Sci. USA 90:8424-8428.
Neumann et al. (1982) EMBO J. 1:841-845.
Nickoloff et al. (1998) Mol. Biotech. 10:93-101.
Notanianni, et al. (1991) J. Reprod. Fert. Suppl., 43:255-260.
O'Gorman et al. (1991) Science 251:1351-55.
Orban et al. (1992) Proc. Nat. Acad. Sci. USA 89:6861-65.
Ory et al. (1996) Proc. Natl. Acad. Sci., USA 93:11400-11406.
Potter et al. (1984) Proc. Natl. Acad. Sci USA 81:7161-65.
Rajewski et al. (1996) J. Clin. Invest. 98:600-03.
Ray and Gage, Biotechniques 13:598-603, 1992.
Reaume, et al. 1996) J. Biol. Chem. 271:23380-88.
Reddy et al. (1991) J. Virol. 65:1507-1515.
Reddy et al. (1992) Proc. Natl. Acad. Sci. U.S.A. 89:6721-25.
Rinchik (1991) Trends in Genetics 7:15-21.
Roach et al. (1995) Exp. Cell Res. 221:520-25.
Robertson, E.J. (1987) Teratocarcinomas and Embryonic Stem Cells: A Practical Approach, Oxford: IRL Press, Washington, D.C..
Robertson et al. (1986) Nature 323:445-48.
Robinson, J. Paul (1997) Current Protocols in Cytometry, John Wiley & Sons, New York, NY.
Russell (1994), Environmental & Molecular Mutagenesis 23(Suppl. 24):23-29.
Sambrook, J. et al. (1989) Molecular Cloning, A Laboratory Manual, Cold Spring Harbor Press, Plainview, NY.
Sauer (1993) Meth. Enz. 225:890-900.
Sauer et al. (1989) Nucleic Acids Research 17:147-61.
Schena et al. (1995-A) Science 270:467-470.
Schena, et al. (1995-B) Proc. Natl. Acad. Sci. (USA) 93:10539-11286.
Schena, M., et al. (1996) Proc. Natl. Acad. Sci. USA 93,10614-10619.
Schnieke, et al. (1997) Science 278:2130-33.
Schwartzberg, et al. (1989) Science 246:799-803.
Sedivy and Sharp (1989) Proc. Natl. Acad. Sci. (USA) 86:227.
Sedivy et al. (1999) T.I.G. 15:88-90.
Shalon, et al. (1996) Genome Res. 6:639-645.
Shesely, et al. (1991) Proc. Natl. Acad. Sci. (USA) 88:4294).
Shiao et al. (1999) Transgenic Res. 8:295-302.
Skarnes et al. (1992) Genes Dev. 6:903-918.
Smith and Berg (1984) Cold Spring Harbor Symp. Quant. Biol. 49:171.
Sokol et al. (1996) Transgenic Research 5:363-71.
St.-Onge et al. (1996) Nucleic Acids Res. 24:3875-77.
Sullivan, et al. (1997) J. Biol. Chem. 272:17972-80.
te Riele et al. (1990) Nature 348:649-651.
Thomas and Capecchi (1987) Cell 51:503-12.
Thomson et al. (1995) Proc. Natl. Acad. Sci. USA 92:7844-7848.
Torres and Kuhn (1997) Laboratory Protocols for Conditional Gene Targeting, Oxford University Press.
Valancius and Smithies (1991) Mol. Cell. Biol. 11:1402.
Van Eyk (2001) Curr Opin Mol Ther 3:546-553.
von Melchner and Ruley (1989) J. Virol. 63:3227-3233.
Waldman and Liskay (1988) Mol. Cell. Biol. 8:5350-5357.
Wilmut, et al. (1997) Nature 385:810.
Yamane et al. (1997) Blood 90:3516-23.
Yoshida et al. (1995) Transgen. Res. 4:277-87).
Zhang et al., Biotechniques 15:868-72, 1993
Zimmer and Gruss (1989) Nature 338:150-153.
Zjilstra et al.(1989) Nature 342:435-438.

PC23222A.ST25.txt
## Claims

1. A genetically-modified non-human mammal, wherein the modification comprises a disrupted REDK gene.

2. The mammal of claim 1, wherein said mammal is a mouse.

3. A genetically-modified animal cell, wherein the modification comprises a disrupted REDK gene.

4. The animal cell of claim 3, wherein said cell is an ES cell or an ES-like cell.

5. The animal cell of claim 3, wherein said cell is isolated from a genetically-modified, non-human mammal of claim 1.

6. The animal cell of claim 3, wherein said cell is a hematopoietic cell or a testicular cell.

7. The animal cell of claim 3, wherein said cell is murine or human.

8. A method of identifying a biological characteristic associated with reduction or elimination of REDK activity comprising comparing a biological characteristic of a genetically-modified, non-human mammal of claim 1, or a genetically-modified animal cell of claim 3, to the characteristic of the appropriate wild-type non-human mammal or animal cell control.

9. A method of identifying a gene that demonstrates modified expression as a result of reduction or elimination of REDK activity, comprising comparing the level of expression of at least one gene other than REDK in an animal cell of claim 3 to the expression of the gene in an appropriate wild-type animal cell control.

10. A method of identifying a protein that demonstrates a modified level or post-translational processing as a result of reduced REDK activity in an animal cell comprising comparing the level or post-translational characteristics of the protein in an animal cell of claim 3 to the level or post-translational characteristics of the protein in an appropriate wild-type animal cell control.
